# EUROPEAN PATENT SPECIFICATION

(11) **EP 3 989 931 B1**
(45) Date of publication and mention of the grant of the patent: **13.12.2023**
(21) Application number: 20735140.4
(22) Date of filing: 24.06.2020
(51) Int. Cl.: A61K 9/00, A24F 47/00, A61K 31/465

(54) **CARBONATED LIQUID NICOTINE FORMULATION**
KARBONISIERTE FLÜSSIGE NIKOTINFORMULIERUNG
FORMULATION DE NICOTINE LIQUIDE GAZEUX

(30) Priority: 25.06.2019 EP 19182407
(43) Date of publication of application: 04.05.2022
(73) Proprietor: Philip Morris Products S.A., 2000 Neuchâtel (CH)
(72) Inventor: EMMETT, Robert, 2000 Neuchâtel (CH); SCHALLER, Jean-Pierre, 2000 Neuchâtel (CH); VUARNOZ-BIZE, Aline, 2000 Neuchâtel (CH)
(74) Representative: Whitfield, Ian
(86) International application number: PCT/EP2020/067747
(87) International publication number: WO 2020/260416

(56) References cited:
- WO-A1-2015/084544
- WO-A2-2018/210681
- US-A1- 2017 079 322
- ORTRUD ASCHENBRENNER ET AL: "Comparative study of solvent properties for carbon dioxide absorption", ENERGY & ENVIRONMENTAL SCIENCE, vol. 3, no. 8, 1 January 2010 (2010-01-01) , page 1106, XP055647525, Cambridge ISSN: 1754-5692, DOI: 10.1039/c002915g

## Description

The invention relates to a carbonated liquid nicotine formulation for use in an aerosol-generating system. The invention also relates to a cartridge for an aerosol-generating system comprising the carbonated liquid nicotine formulation and an aerosol-generating system comprising the carbonated liquid nicotine formulation and an atomiser configured to generate an aerosol from the carbonated liquid nicotine formulation.

Aerosol-generating systems for delivering nicotine to a user that comprise an atomiser configured to generate an inhalable aerosol from a liquid nicotine formulation are known. Some known aerosol-generating systems comprise a thermal atomiser such as an electric heater that is configured to heat and vaporise the liquid nicotine formulation to generate an aerosol. Other known aerosol-generating systems comprise a non-thermal atomiser that is configured to generate an aerosol from the liquid nicotine formulation using, for example, impinging jet, ultrasonic or vibrating mesh technologies. Typical liquid nicotine formulations for use in aerosol-generating systems comprise glycerine, propylene glycol and water as solvents.

WO 2018/210681 A2 describes a liquid tobacco extract and the use of a liquid tobacco extract in a device for generating an inhalable medium. The liquid tobacco extract contains tobacco components dissolved in a solvent. The solvent may comprise or consist of one or more polyols, suitably propylene glycol and/or glycerol. The method of making the liquid tobacco extract comprises (i) extraction of tobacco components from tobacco using a supercritical extraction solvent, and (ii) transfer of the extracted tobacco components into a liquid solvent. The supercritical extraction solvent may be carbon dioxide. The liquid tobacco extract may have a water activity of less than about 0.45 Aw at 25°C.

It would be desirable to provide a liquid nicotine formulation for use in an aerosol-generating system that that enables generation of an aerosol providing improved nicotine satisfaction to a user compared to such typical liquid nicotine formulations.

According to the invention there is provided a carbonated liquid nicotine formulation for use in an aerosol-generating system, the carbonated liquid nicotine formulation comprising at least one of water and one or more water-miscible solvents, wherein the carbonated liquid formulation has an ethanol content of less than 1 percent by weight, a nicotine content of greater than or equal to 0.25 percent by weight and a carbon dioxide content of greater than or equal to 0.5 percent by weight.

According to the invention there is also provided a cartridge for use in an aerosol-generating system, the cartridge containing a carbonated liquid nicotine formulation comprising at least one of water and one or more water-miscible solvents, wherein the carbonated liquid formulation has an ethanol content of less than 1 percent by weight, a nicotine content of greater than or equal to 0.25 percent by weight and a carbon dioxide content of greater than or equal to 0.5 percent by weight.

According to the invention there is further provided an aerosol-generating system comprising: a carbonated liquid nicotine formulation comprising at least one of water and one or more water-miscible solvents, wherein the carbonated liquid formulation has an ethanol content of less than 1 percent by weight, a nicotine content of greater than or equal to 0.25 percent by weight and a carbon dioxide content of greater than or equal to 0.5 percent by weight; and an atomiser configured to generate an aerosol from the carbonated liquid nicotine formulation.

As used herein with reference to the invention, the term "liquid nicotine formulation" describes a liquid formulation comprising nicotine.

As used herein with reference to the invention, the term "carbonated liquid nicotine formulation" describes a liquid formulation comprising nicotine that has been impregnated with carbon dioxide gas under pressure.

As used herein with reference to the invention, the term "nicotine" describes nicotine, nicotine base or a nicotine salt. In embodiments in which the liquid nicotine formulation comprises a nicotine base or a nicotine salt, the amounts of nicotine recited herein are the amount of free base nicotine or amount of protonated nicotine, respectively.

As used herein with reference to the invention, the term "water-miscible solvent" describes a compound other than water or an organic acid that is liquid at 20°C and mixes with water in all proportions to form a homogenous solution.

Carbonation of the substantially ethanol-free liquid nicotine formulation according to the invention advantageously enables generation of an inhalable aerosol providing improved nicotine satisfaction to a user compared to aerosols generated from typical liquid nicotine formulations.

As used herein with reference to the invention, the term "substantially ethanol-free liquid nicotine formulation" describes a liquid formulation having an ethanol content of less than 1 percent by weight.

In particular, inclusion of greater than or equal to about 0.5 percent by weight carbon dioxide in the substantially ethanol-free, carbonated liquid nicotine formulation advantageously enables generation of an inhalable aerosol providing increased nicotine delivery to a user compared to aerosols generated from typical liquid nicotine formulations.

Inclusion of greater than or equal to about 0.5 percent by weight carbon dioxide in the substantially ethanol-free, carbonated liquid nicotine formulation advantageously enables generation of an inhalable aerosol providing increased delivery of non-polar components of the carbonated liquid nicotine formulation to a user compared to aerosols generated from typical liquid nicotine formulations.

For example, where the substantially ethanol-free, carbonated liquid nicotine formulation comprises one or more non-polar flavourants, inclusion of greater than or equal to about 0.5 percent by weight carbon dioxide in the substantially ethanol-free, carbonated liquid nicotine formulation advantageously enables generation of an inhalable aerosol providing increased non-polar flavourant delivery to a user compared to aerosols generated from typical liquid nicotine formulations.

Carbonation of the substantially ethanol-free liquid nicotine formulation according to the invention advantageously enables generation of an inhalable aerosol providing an enhanced sensory perception to a user compared to aerosols generated from typical liquid nicotine formulations. In particular, carbonation of the substantially ethanol-free liquid nicotine formulation according to the invention advantageously enables generation of an inhalable aerosol providing increased oral sensation to a user compared to aerosols generated from typical liquid nicotine formulations.

Unless stated otherwise, percentages by weight of components of the carbonated liquid nicotine formulation recited herein are based on the total weight of the carbonated liquid nicotine formulation.

The carbonated liquid nicotine formulation has an ethanol content of less than 1 percent by weight.

Preferably, the carbonated liquid nicotine formulation has an ethanol content of less than or equal to about 0.75 percent by weight. More preferably, the carbonated liquid nicotine formulation has an ethanol content of less than or equal to about 0.5 percent by weight. Most preferably, the carbonated liquid nicotine formulation has an ethanol content of less than or equal to about 0.25 percent by weight

The carbonated liquid nicotine formulation may have an ethanol content of less than or equal to about 0.2 percent by weight. For example, the carbonated liquid nicotine formulation may have an ethanol content of less than about 0.15 percent by weight, less than about 0.1 percent by weight or less than about 0.05 percent by weight.

The carbonated liquid nicotine formulation may be an ethanol-free carbonated liquid nicotine formulation.

As used herein with reference to the invention, the term "ethanol-free carbonated liquid nicotine formulation" describes a carbonated liquid formulation having an ethanol content of 0 percent by weight.

The carbonated liquid nicotine formulation may be a substantially monohydric alcohol-free carbonated liquid nicotine formulation.

As used herein with reference to the invention, the term "substantially monohydric alcohol-free liquid nicotine formulation" describes a liquid formulation having a monohydric alcohol content of less than 1 percent by weight.

Preferably, the carbonated liquid nicotine formulation has a monohydric alcohol of less than or equal to about 0.75 percent by weight. More preferably, the carbonated liquid nicotine formulation has a monohydric alcohol content of less than or equal to about 0.5 percent by weight. Most preferably, the carbonated liquid nicotine formulation has a monohydric alcohol content of less than or equal to about 0.25 percent by weight.

The carbonated liquid nicotine formulation may have a monohydric alcohol content of less than or equal to about 0.2 percent by weight. For example, the carbonated liquid nicotine formulation may have a monohydric alcohol content of less than about 0.15 percent by weight, less than about 0.1 percent by weight or less than about 0.05 percent by weight.

The carbonated liquid nicotine formulation may be a monohydric alcohol-free carbonated liquid nicotine formulation.

As used herein with reference to the invention, the term "monohydric alcohol-free carbonated liquid nicotine formulation" describes a carbonated liquid formulation having a monohydric alcohol content of 0 percent by weight.

The carbonated liquid nicotine formulation may comprise natural nicotine or synthetic nicotine.

The carbonated liquid nicotine formulation has a nicotine content of greater than or equal to about 0.25 percent by weight.

The liquid nicotine formulation may have a nicotine content of greater than or equal to about 0.5 percent by weight.

Preferably, the carbonated liquid nicotine formulation has a nicotine content of greater than or equal to about 0.75 percent by weight. More preferably, the carbonated liquid nicotine formulation has a nicotine content of greater than or equal to about 1 percent by weight.

The liquid nicotine formulation may have a nicotine content of greater than or equal to about 1.5 percent by weight.

The carbonated liquid nicotine formulation may have a nicotine content of less than or equal to about 10 percent by weight. The carbonated liquid nicotine formulation may have a nicotine content of less than or equal to about 8 percent by weight.

Preferably, the carbonated liquid nicotine formulation has a nicotine content of less than or equal to about 5 percent by weight. More preferably, the carbonated liquid nicotine formulation has a nicotine content of less than or equal to about 3 percent by weight.

The carbonated liquid nicotine formulation may have a nicotine content of between about 0.5 percent by weight and about 10 percent by weight. For example, the carbonated liquid nicotine formulation may have a nicotine content of between about 0.5 percent by weight and about 8 percent by weight, between about 0.5 percent by weight and about 5 percent by weight or between about 0.5 percent by weight and about 3 percent by weight.

Preferably, the carbonated liquid nicotine formulation has a nicotine content of between about 0.75 percent by weight and about 10 percent by weight. For example, the carbonated liquid nicotine formulation may have a nicotine content of between about 0.75 percent by weight and about 8 percent by weight, between about 0.75 percent by weight and about 5 percent by weight or between about 0.75 percent by weight and about 3 percent by weight.

More preferably, the carbonated liquid nicotine formulation has a nicotine content of between about 1 percent by weight and about 10 percent by weight. For example, the carbonated liquid nicotine formulation may have a nicotine content of between about 1 percent by weight and about 8 percent by weight, between about 1 percent by weight and about 5 percent by weight or between about 1 percent by weight and about 3 percent by weight.

The carbonated liquid nicotine formulation may have a nicotine content of between about 1.5 percent by weight and about 10 percent by weight. For example, the carbonated liquid nicotine formulation may have a nicotine content of between about 1.5 percent by weight and about 8 percent by weight, between about 1.5 percent by weight and about 5 percent by weight or between about 1.5 percent by weight and about 3 percent by weight.

The ratio of the weight percent ethanol content to the weight percent nicotine content of the carbonated liquid nicotine formulation may be less than or equal to about 3 or less than or equal to 2.

Preferably, the ratio of the weight percent ethanol content to the weight percent nicotine content of the carbonated liquid nicotine formulation is less than or equal to about 1. For example, the ratio of the weight percent ethanol content to the weight percent nicotine content of the carbonated liquid nicotine formulation may be less than or equal to about 0.75, less than or equal to about 0.5 or less than or equal to about 0.25.

The ratio of the weight percent monohydric alcohol content to the weight percent nicotine content of the carbonated liquid nicotine formulation may be less than or equal to about 3 or less than or equal to 2.

Preferably, the ratio of the weight percent monohydric alcohol content to the weight percent nicotine content of the carbonated liquid nicotine formulation is less than or equal to about 1. For example, the ratio of the weight percent monohydric alcohol content to the weight percent nicotine content of the carbonated liquid nicotine formulation may be less than or equal to about 0.75, less than or equal to about 0.5 or less than or equal to about 0.25.

The carbonated liquid nicotine formulation has a carbon dioxide content of greater than or equal to about 0.5 percent by weight.

Preferably, the carbonated liquid nicotine formulation has a carbon dioxide content of greater than or equal to about 0.75 percent by weight. More preferably, the carbonated liquid nicotine formulation has a carbon dioxide content of greater than or equal to about 1 percent by weight. Most preferably, the carbonated liquid nicotine formulation has a carbon dioxide content of greater than or equal to about 1.25 percent by weight.

The carbonated liquid nicotine formulation may have a carbon dioxide content of less than or equal to about 8 percent by weight. The carbonated liquid nicotine formulation may have a carbon dioxide content of less than or equal to about 7 percent by weight or less than or equal to about 6 percent by weight.

Preferably, the carbonated liquid nicotine formulation has a carbon dioxide content of less than or equal to about 5 percent by weight. More preferably, the carbonated liquid nicotine formulation has a carbon dioxide content of less than or equal to about 4 percent by weight. Most preferably, the carbonated liquid nicotine formulation has a carbon dioxide content of less than or equal to about 3 percent by weight.

The carbonated liquid nicotine formulation may have a carbon dioxide content of between about 0.5 percent by weight and about 8 percent by weight. For example, the carbonated liquid nicotine formulation may have a carbon dioxide content of between about 0.5 percent by weight and about 7 percent by weight, between about 0.5 percent by weight and about 6 percent by weight, between about 0.5 percent by weight and about 5 percent by weight, between about 0.5 percent by weight and about 4 percent by weight or between about 0.5 percent by weight and about 3 percent by weight.

Preferably, the carbonated liquid nicotine formulation has a carbon dioxide content of between about 0.75 percent by weight and about 8 percent by weight. For example, the carbonated liquid nicotine formulation may have a carbon dioxide content of between about 0.75 percent by weight and about 7 percent by weight, between about 0.75 percent by weight and about 6 percent by weight, between about 0.75 percent by weight and about 5 percent by weight, between about 0.75 percent by weight and about 4 percent by weight or between about 0.75 percent by weight and about 3 percent by weight.

More preferably, the carbonated liquid nicotine formulation has a carbon dioxide content of between about 1 percent by weight and about 8 percent by weight. For example, the carbonated liquid nicotine formulation may have a carbon dioxide content of between about 1 percent by weight and about 7 percent by weight, between about 1 percent by weight and about 6 percent by weight, between about 1 percent by weight and about 5 percent by weight, between about 1 percent by weight and about 4 percent by weight or between about 1 percent by weight and about 3 percent by weight.

Most preferably, the carbonated liquid nicotine formulation has a carbon dioxide content of between about 1.25 percent by weight and about 8 percent by weight. For example, the carbonated liquid nicotine formulation may have a carbon dioxide content of between about 1.25 percent by weight and about 7 percent by weight, between about 1.25 percent by weight and about 6 percent by weight, between about 1.25 percent by weight and about 5 percent by weight, between about 1.25 percent by weight and about 4 percent by weight or between about 1.25 percent by weight and about 3 percent by weight.

The ratio of the weight percent ethanol content to the weight percent carbon dioxide content of the carbonated liquid nicotine formulation may be less than or equal to about 1.5 or less than or equal to 1.25.

Preferably, the ratio of the weight percent ethanol content to the weight percent carbon dioxide content of the carbonated liquid nicotine formulation is less than or equal to about 1. For example, the ratio of the weight percent ethanol content to the weight percent carbon dioxide content of the carbonated liquid nicotine formulation may be less than or equal to about 0.75, less than or equal to about 0.5 or less than or equal to about 0.25.

The ratio of the weight percent monohydric alcohol content to the weight percent carbon dioxide content of the carbonated liquid nicotine formulation may be less than or equal to about 1.5 or less than or equal to 1.25.

Preferably, the ratio of the weight percent monohydric alcohol content to the weight percent carbon dioxide content of the carbonated liquid nicotine formulation is less than or equal to about 1. For example, the ratio of the weight percent monohydric alcohol content to the weight percent carbon dioxide content of the carbonated liquid nicotine formulation may be less than or equal to about 0.75, less than or equal to about 0.5 or less than or equal to about 0.25.

The ratio of the weight percent carbon dioxide content to the weight percent nicotine content of the carbonated liquid nicotine formulation may be greater than or equal to about 1. For example, the ratio of the weight percent carbon dioxide content to the weight percent nicotine content of the carbonated liquid nicotine formulation may be greater than or equal to about 1.25.

The ratio of the weight percent carbon dioxide content to the weight percent nicotine content of the carbonated liquid nicotine formulation may be less than or equal to about 2. For example, the ratio of the weight percent carbon dioxide content to the weight percent nicotine content of the carbonated liquid nicotine formulation may be less than or equal to about 1.75.

The ratio of the weight percent carbon dioxide content to the weight percent nicotine content of the carbonated liquid nicotine formulation may be between about 1 and about 2. For example, the ratio of the weight percent carbon dioxide content to the weight percent nicotine content of the carbonated liquid nicotine formulation may be between about 1 and about 1.75.

The ratio of the weight percent carbon dioxide content to the weight percent nicotine content of the carbonated liquid nicotine formulation may be between about 1.25 and about 2. For example, the ratio of the weight percent carbon dioxide content to the weight percent nicotine content of the carbonated liquid nicotine formulation may be between about 1.25 and about 1.75.

The carbonated liquid nicotine formulation may be carbonated using suitable known methods and apparatus.

The carbonated liquid nicotine formulation comprises at least one of water and one or more water-miscible solvents. That is, the carbonated liquid nicotine comprises water or one or more miscible solvents or both water and one or more miscible solvents.

Preferably, the carbonated liquid nicotine formulation has a total water and water-miscible solvent content of greater than or equal to about 70 percent by weight.

More preferably, the carbonated liquid nicotine formulation has a total water and water-miscible solvent content of greater than or equal to about 75 percent by weight. For example, the carbonated liquid nicotine formulation may have a total water and water-miscible solvent content of greater than or equal to 80 percent by weight or greater than or equal to about 85 percent by weight.

In embodiments in which the carbonated liquid nicotine formulation comprises water, the water may advantageously help to dissolve and stabilise polar components and ionic components of the carbonated liquid nicotine formulation.

In embodiments in which the carbonated liquid nicotine formulation comprises water, preferably the carbonated liquid nicotine formulation has a water content of greater than or equal to about 5 percent by weight. More preferably, the carbonated liquid nicotine formulation has a water-content of greater than or equal to about 10 percent by weight. For example, the carbonated liquid nicotine formulation may have a water content of greater than or equal to about 15 percent by weight or greater than or equal to about 20 percent by weight.

In embodiments in which the carbonated liquid nicotine formulation comprises water, preferably the carbonated liquid nicotine formulation has a water content of less than or equal to about 80 percent by weight. More preferably, the carbonated liquid nicotine formulation has a water content of less than or equal to about 75 percent by weight. For example, the carbonated liquid nicotine formulation may have a water content of less than or equal to about 70 percent by weight or less than or equal to about 65 percent by weight.

In embodiments in which the carbonated liquid nicotine formulation comprises water, preferably the carbonated liquid nicotine formulation has a water content of between about 5 percent by weight and about 80 percent by weight. For example, the carbonated liquid nicotine formulation may have a water content of between about 5 percent by weight and about 75 percent by weight, between about 5 percent by weight and about 70 percent by weight or between about 5 percent by weight and about 65 percent by weight.

More preferably, the carbonated liquid nicotine formulation has a water content of between about 10 percent by weight and about 80 percent by weight. For example, the carbonated liquid nicotine formulation may have a water content of between about 10 percent by weight and about 75 percent by weight, between about 10 percent by weight and about 70 percent by weight or between about 10 percent by weight and about 65 percent by weight.

In embodiments in which the carbonated liquid nicotine formulation comprises water, the carbonated liquid nicotine formulation may have a water content of between about 15 percent by weight and about 80 percent by weight. For example, the carbonated liquid nicotine formulation may have a water content of between about 15 percent by weight and about 75 percent by weight, between about 15 percent by weight and about 70 percent by weight or between about 15 percent by weight and about 65 percent by weight.

The carbonated liquid nicotine formulation may have a water content of between about 20 percent by weight and about 80 percent by weight. For example, the carbonated liquid nicotine formulation may have a water content of between about 20 percent by weight and about 75 percent by weight, between about 20 percent by weight and about 70 percent by weight or between about 20 percent by weight and about 65 percent by weight.

In embodiments in which the carbonated liquid nicotine formulation comprises one or more water-miscible solvents, the one or more water-miscible solvents may advantageously help to dissolve and stabilise polar components and ionic components of the carbonated liquid nicotine formulation.

In embodiments in which the carbonated liquid nicotine formulation comprises one or more water-miscible solvents, preferably the carbonated liquid nicotine formulation has a water-miscible solvent content of greater than or equal to about 5 percent by weight. More preferably, the carbonated liquid nicotine formulation has a water-miscible solvent content of greater than or equal to about 10 percent by weight. For example, the carbonated liquid nicotine formulation may have a water-miscible solvent content of greater than or equal to about 15 percent by weight, greater than or equal to about 20 percent by weight or greater than or equal to about 25 percent by weight.

In embodiments in which the carbonated liquid nicotine formulation comprises one or more water-miscible solvents, preferably the carbonated liquid nicotine formulation has a water-miscible solvent content of less than or equal to about 85 percent by weight. More preferably, the carbonated liquid nicotine formulation has a water-miscible solvent content of less than or equal to about 80 percent by weight. For example, the carbonated liquid nicotine formulation may have a water-miscible solvent content of less than or equal to about 75 percent by weight or less than or equal to about 70 percent by weight.

In embodiments in which the carbonated liquid nicotine formulation comprises one or more water-miscible solvents, preferably the carbonated liquid nicotine formulation has a water-miscible solvent content of between about 5 percent by weight and about 85 percent by weight. For example, the carbonated liquid nicotine formulation may have a water-miscible solvent content of between about 5 percent by weight and about 80 percent by weight, between about 5 percent by weight and about 75 percent by weight or between about 5 percent by weight and about 70 percent by weight.

More preferably, the carbonated liquid nicotine formulation has a water-miscible solvent content of between about 10 percent by weight and about 85 percent by weight. For example, the carbonated liquid nicotine formulation may have a water-miscible solvent content of between about 10 percent by weight and about 80 percent by weight, between about 10 percent by weight and about 75 percent by weight or between about 10 percent by weight and about 70 percent by weight.

In embodiments in which the carbonated liquid nicotine formulation comprises one or more water-miscible solvents, the carbonated liquid nicotine formulation may have a water-miscible solvent content of between about 15 percent by weight and about 85 percent by weight, between about 15 percent by weight and about 80 percent by weight, between about 15 percent by weight and about 75 percent by weight or between about 15 percent by weight and about 70 percent by weight.

The carbonated liquid nicotine formulation may have a water-miscible solvent content of between about 20 percent by weight and about 85 percent by weight, between about 20 percent by weight and about 80 percent by weight, between about 20 percent by weight and about 75 percent by weight or between about 20 percent by weight and about 70 percent by weight.

The carbonated liquid nicotine formulation may have a water-miscible solvent content of between about 25 percent by weight and about 85 percent by weight, between about 25 percent by weight and about 80 percent by weight, between about 25 percent by weight and about 75 percent by weight or between about 25 percent by weight and about 70 percent by weight.

In embodiments in which the carbonated liquid nicotine formulation comprises one or more water-miscible solvents, preferably, the one or more water-miscible solvents are one or more water-miscible polyhydric alcohols.

As used herein with reference to the invention, the term "water-miscible polyhydric alcohol" describes a polyhydric alcohol that is liquid at 20°C and mixes with water in all proportions to form a homogenous solution.

According to a preferred embodiment of the invention there is provided a carbonated liquid nicotine formulation for use in an aerosol-generating system, the carbonated liquid nicotine formulation comprising at least one of water and one or more water-miscible polyhydric alcohols, wherein the carbonated liquid formulation has an ethanol content of less than 1 percent by weight, a nicotine content of greater than or equal to 0.25 percent by weight and a carbon dioxide content of greater than or equal to 0.5 percent by weight.

According to a preferred embodiment of the invention there is also provided a cartridge for use in an aerosol-generating system, the cartridge containing a carbonated liquid nicotine formulation comprising at least one of water and one or more water-miscible polyhydric alcohols, wherein the carbonated liquid formulation has an ethanol content of less than 1 percent by weight, a nicotine content of greater than or equal to 0.25 percent by weight and a carbon dioxide content of greater than or equal to 0.5 percent by weight.

According to a preferred embodiment of the invention there is further provided an aerosol-generating system comprising: a carbonated liquid nicotine formulation comprising at least one of water and one or more water-miscible polyhydric alcohols, wherein the carbonated liquid formulation has an ethanol content of less than 1 percent by weight, a nicotine content of greater than or equal to 0.25 percent by weight and a carbon dioxide content of greater than or equal to 0.5 percent by weight; and an atomiser configured to generate an aerosol from the carbonated liquid nicotine formulation.

In embodiments in which the carbonated liquid nicotine formulation comprises one or more water-miscible polyhydric alcohols, preferably the carbonated liquid nicotine formulation has a water-miscible polyhydric alcohol content of greater than or equal to about 5 percent by weight. More preferably, the carbonated liquid nicotine formulation has a water-miscible polyhydric alcohol content of greater than or equal to about 10 percent by weight. For example, the carbonated liquid nicotine formulation may have a water-miscible polyhydric alcohol content of greater than or equal to about 15 percent by weight, greater than or equal to about 20 percent by weight or greater than or equal to about 25 percent by weight.

In embodiments in which the carbonated liquid nicotine formulation comprises one or more water-miscible polyhydric alcohols, preferably the carbonated liquid nicotine formulation has a water-miscible polyhydric alcohol content of less than or equal to about 85 percent by weight. More preferably, the carbonated liquid nicotine formulation has a water-miscible polyhydric alcohol content of less than or equal to about 80 percent by weight. For example, the carbonated liquid nicotine formulation may have a water-miscible polyhydric alcohol content of less than or equal to about 75 percent by weight or less than or equal to about 70 percent by weight.

In embodiments in which the carbonated liquid nicotine formulation comprises one or more water-miscible polyhydric alcohols, preferably the carbonated liquid nicotine formulation has a water-miscible polyhydric alcohol content of between about 5 percent by weight and about 85 percent by weight. For example, the carbonated liquid nicotine formulation may have a water-miscible polyhydric alcohol content of between about 5 percent by weight and about 80 percent by weight, between about 5 percent by weight and about 75 percent by weight or between about 5 percent by weight and about 70 percent by weight.

More preferably, the carbonated liquid nicotine formulation has a water-miscible polyhydric alcohol content of between about 10 percent by weight and about 85 percent by weight. For example, the carbonated liquid nicotine formulation may have a water-miscible polyhydric alcohol content of between about 10 percent by weight and about 80 percent by weight, between about 10 percent by weight and about 75 percent by weight or between about 10 percent by weight and about 70 percent by weight.

In embodiments in which the carbonated liquid nicotine formulation comprises one or more water-miscible polyhydric alcohols, the carbonated liquid nicotine formulation may have a water-miscible polyhydric alcohol content of between about 15 percent by weight and about 85 percent by weight, between about 15 percent by weight and about 80 percent by weight, between about 15 percent by weight and about 75 percent by weight or between about 15 percent by weight and about 70 percent by weight.

The carbonated liquid nicotine formulation may have a water-miscible polyhydric alcohol content of between about 20 percent by weight and about 85 percent by weight, between about 20 percent by weight and about 80 percent by weight, between about 20 percent by weight and about 75 percent by weight or between about 20 percent by weight and about 70 percent by weight.

The carbonated liquid nicotine formulation may have a water-miscible solvent polyhydric alcohol content of between about 25 percent by weight and about 85 percent by weight, between about 25 percent by weight and about 80 percent by weight, between about 25 percent by weight and about 75 percent by weight or between about 25 percent by weight and about 70 percent by weight.

In embodiments in which the carbonated liquid nicotine formulation comprises one or more water-miscible polyhydric alcohols, preferably the one or more water-miscible polyhydric alcohols are selected from the group consisting of 1,3-butanediol, glycerine, propylene glycol, and triethylene glycol.

More preferably, the one or more water-miscible polyhydric alcohols are selected from the group consisting of glycerine and propylene glycol.

Most preferably, the one or more water-miscible polyhydric alcohols are selected from the group consisting of vegetable glycerine and propylene glycol.

The carbonated liquid nicotine formulation may comprise glycerine and propylene glycol.

Preferably, the ratio of the weight percent glycerine content to the weight percent propylene glycol content of the carbonated liquid nicotine formulation is 1.

Increasing the proportion of propylene glycol in the carbonated liquid nicotine formulation may improve the ability of the carbonated liquid nicotine formulation to absorb flavours.

Increasing the proportion of glycerine in the carbonated liquid nicotine formulation may increase the volume of aerosol generated from the carbonated liquid nicotine formulation.

The ratio of the weight percent glycerine content to the weight percent propylene glycol content of the carbonated liquid nicotine formulation may be less than or equal to 1.25. The ratio of the weight percent glycerine content to the weight percent propylene glycol content of the carbonated liquid nicotine formulation may be less than or equal to 1.2. The ratio of the weight percent glycerine content to the weight percent propylene glycol content of the carbonated liquid nicotine formulation may be less than or equal to 1.1. The ratio of the weight percent glycerine content to the weight percent propylene glycol content of the carbonated liquid nicotine formulation may be less than or equal to 1. The ratio of the weight percent glycerine content to the weight percent propylene glycol content of the carbonated liquid nicotine formulation may be less than or equal to 0.9. The ratio of the weight percent glycerine content to the weight percent propylene glycol content of the carbonated liquid nicotine formulation may be less than or equal to 0.8.

The ratio of the weight percent glycerine content to the weight percent propylene glycol content of the carbonated liquid nicotine formulation may be greater than or equal to 0.75. The ratio of the weight percent glycerine content to the weight percent propylene glycol content of the carbonated liquid nicotine formulation may be greater than or equal to 0.8. The ratio of the weight percent glycerine content to the weight percent propylene glycol content of the carbonated liquid nicotine formulation may be greater than or equal to 0.9. The ratio of the weight percent glycerine content to the weight percent propylene glycol content of the carbonated liquid nicotine formulation may be greater than or equal to 1. The ratio of the weight percent glycerine content to the weight percent propylene glycol content of the carbonated liquid nicotine formulation may be greater than or equal to 1.1. The ratio of the weight percent glycerine content to the weight percent propylene glycol content of the carbonated liquid nicotine formulation may be greater than or equal to 1.2. The ratio of the weight percent glycerine content to the weight percent propylene glycol content of the carbonated liquid nicotine formulation may be greater than or equal to 1.25.

The ratio of the weight percent glycerine content to the weight percent propylene glycol content of the carbonated liquid nicotine formulation may be between 0.75 and 1.25. The ratio of the weight percent glycerine content to the weight percent propylene glycol content of the carbonated liquid nicotine formulation may be between 0.8 and 1.2. The ratio of the weight percent glycerine content to the weight percent propylene glycol content of the carbonated liquid nicotine formulation may be between 0.8 and 1.1. The ratio of the weight percent glycerine content to the weight percent propylene glycol content of the carbonated liquid nicotine formulation may be between 0.9 and 1.1. The ratio of the weight percent glycerine content to the weight percent propylene glycol content of the carbonated liquid nicotine formulation may be between 0.9 and 1. The ratio of the weight percent glycerine content to the weight percent propylene glycol content of the carbonated liquid nicotine formulation may be between 1 and 1.1.

According to a preferred embodiment of the invention there is provided a carbonated liquid nicotine formulation for use in an aerosol-generating system, the carbonated liquid nicotine formulation comprising at least one of water and one or more water-miscible polyhydric alcohols selected from the group consisting of glycerine and propylene glycol, wherein the carbonated liquid formulation has an ethanol content of less than 1 percent by weight, a nicotine content of greater than or equal to 0.25 percent by weight and a carbon dioxide content of greater than or equal to 0.5 percent by weight.

According to a preferred embodiment of the invention there is also provided a cartridge for use in an aerosol-generating system, the cartridge containing a carbonated liquid nicotine formulation comprising at least one of water and one or more water-miscible polyhydric alcohols selected from the group consisting of glycerine and propylene glycol, wherein the carbonated liquid formulation has an ethanol content of less than 1 percent by weight, a nicotine content of greater than or equal to 0.25 percent by weight and a carbon dioxide content of greater than or equal to 0.5 percent by weight.

According to a preferred embodiment of the invention there is further provided an aerosol-generating system comprising: a carbonated liquid nicotine formulation comprising at least one of water and one or more water-miscible polyhydric alcohols selected from the group consisting of glycerine and propylene glycol, wherein the carbonated liquid formulation has an ethanol content of less than 1 percent by weight, a nicotine content of greater than or equal to 0.25 percent by weight and a carbon dioxide content of greater than or equal to 0.5 percent by weight; and an atomiser configured to generate an aerosol from the carbonated liquid nicotine formulation.

In embodiments in which the carbonated liquid nicotine formulation comprises one or more water-miscible polyhydric alcohols selected from the group consisting of glycerine and propylene glycol, preferably the carbonated liquid nicotine formulation has a combined glycerine and propylene glycol content of greater than or equal to about 5 percent by weight. More preferably, the carbonated liquid nicotine formulation has a combined glycerine and propylene glycol content of greater than or equal to about 10 percent by weight. For example, the carbonated liquid nicotine formulation may have a combined glycerine and propylene glycol content of greater than or equal to about 15 percent by weight, greater than or equal to about 20 percent by weight or greater than or equal to about 25 percent by weight.

In embodiments in which the carbonated liquid nicotine formulation comprises one or more water-miscible polyhydric alcohols selected from the group consisting of glycerine and propylene glycol, preferably the carbonated liquid nicotine formulation has a combined glycerine and propylene glycol content of less than or equal to about 85 percent by weight. More preferably, the carbonated liquid nicotine formulation has a combined glycerine and propylene glycol content of less than or equal to about 80 percent by weight. For example, the carbonated liquid nicotine formulation may have a combined glycerine and propylene glycol content of less than or equal to about 75 percent by weight or less than or equal to about 70 percent by weight.

In embodiments in which the carbonated liquid nicotine formulation comprises one or more water-miscible polyhydric alcohols selected from the group consisting of glycerine and propylene glycol, preferably the carbonated liquid nicotine formulation has a combined glycerine and propylene glycol content of between about 5 percent by weight and about 85 percent by weight. For example, the carbonated liquid nicotine formulation may have a combined glycerine and propylene glycol content of between about 5 percent by weight and about 80 percent by weight, between about 5 percent by weight and about 75 percent by weight or between about 5 percent by weight and about 70 percent by weight.

More preferably, the carbonated liquid nicotine formulation has a combined glycerine and propylene glycol content of between about 10 percent by weight and about 85 percent by weight. For example, the carbonated liquid nicotine formulation may have a combined glycerine and propylene glycol content of between about 10 percent by weight and about 80 percent by weight, between about 10 percent by weight and about 75 percent by weight or between about 10 percent by weight and about 70 percent by weight.

In embodiments in which the carbonated liquid nicotine formulation comprises one or more water-miscible polyhydric alcohols selected from the group consisting of glycerine and propylene glycol, the carbonated liquid nicotine formulation may have a combined glycerine and propylene glycol content of between about 15 percent by weight and about 85 percent by weight, between about 15 percent by weight and about 80 percent by weight, between about 15 percent by weight and about 75 percent by weight or between about 15 percent by weight and about 70 percent by weight.

The carbonated liquid nicotine formulation may have a combined glycerine and propylene glycol content of between about 20 percent by weight and about 85 percent by weight, between about 20 percent by weight and about 80 percent by weight, between about 20 percent by weight and about 75 percent by weight or between about 20 percent by weight and about 70 percent by weight.

The carbonated liquid nicotine formulation may have a combined glycerine and propylene glycol content of between about 25 percent by weight and about 85 percent by weight, between about 25 percent by weight and about 80 percent by weight, between about 25 percent by weight and about 75 percent by weight or between about 25 percent by weight and about 70 percent by weight.

Preferably, the carbonated liquid nicotine formulation comprises water and one or more water-miscible solvents.

According to a preferred embodiment of the invention there is provided a carbonated liquid nicotine formulation for use in an aerosol-generating system, the carbonated liquid nicotine formulation comprising water and one or more water-miscible solvents, wherein the carbonated liquid formulation has an ethanol content of less than 1 percent by weight, a nicotine content of greater than or equal to 0.25 percent by weight and a carbon dioxide content of greater than or equal to 0.5 percent by weight.

According to a preferred embodiment of the invention there is also provided a cartridge for use in an aerosol-generating system, the cartridge containing a carbonated liquid nicotine formulation comprising water and one or more water-miscible solvents, wherein the carbonated liquid formulation has an ethanol content of less than 1 percent by weight, a nicotine content of greater than or equal to 0.25 percent by weight and a carbon dioxide content of greater than or equal to 0.5 percent by weight.

According to a preferred embodiment of the invention there is further provided an aerosol-generating system comprising: a carbonated liquid nicotine formulation comprising water and one or more water-miscible solvents, wherein the carbonated liquid formulation has an ethanol content of less than 1 percent by weight, a nicotine content of greater than or equal to 0.25 percent by weight and a carbon dioxide content of greater than or equal to 0.5 percent by weight; and an atomiser configured to generate an aerosol from the carbonated liquid nicotine formulation.

In embodiments in which the carbonated liquid nicotine formulation comprises water and one or more water-miscible solvents, preferably the carbonated liquid nicotine formulation has a combined water and water-miscible solvent content of greater than or equal to about 70 percent by weight. More preferably, the carbonated liquid nicotine formulation has a combined water and water-miscible solvent content of greater than or equal to 75 percent by weight. For example, the carbonated liquid nicotine formulation may have a combined water and water-miscible solvent content of greater than or equal to about 80 percent by weight or greater than or equal to about 85 percent by weight.

More preferably, the carbonated liquid nicotine formulation comprises water and one or more water-miscible polyhydric alcohols.

According to a more preferred embodiment of the invention there is provided a carbonated liquid nicotine formulation for use in an aerosol-generating system, the carbonated liquid nicotine formulation comprising water and one or more water-miscible polyhydric alcohols, wherein the carbonated liquid formulation has an ethanol content of less than 1 percent by weight, a nicotine content of greater than or equal to 0.25 percent by weight and a carbon dioxide content of greater than or equal to 0.5 percent by weight.

According to a more preferred embodiment of the invention there is also provided a cartridge for use in an aerosol-generating system, the cartridge containing a carbonated liquid nicotine formulation comprising water and one or more water-miscible polyhydric alcohols, wherein the carbonated liquid formulation has an ethanol content of less than 1 percent by weight, a nicotine content of greater than or equal to 0.25 percent by weight and a carbon dioxide content of greater than or equal to 0.5 percent by weight.

According to a more preferred embodiment of the invention there is further provided an aerosol-generating system comprising: a carbonated liquid nicotine formulation comprising water and one or more water-miscible polyhydric alcohols, wherein the carbonated liquid formulation has an ethanol content of less than 1 percent by weight, a nicotine content of greater than or equal to 0.25 percent by weight and a carbon dioxide content of greater than or equal to 0.5 percent by weight; and an atomiser configured to generate an aerosol from the carbonated liquid nicotine formulation.

In embodiments in which the carbonated liquid nicotine formulation comprises water and one or more water-miscible polyhydric alcohols, preferably the carbonated liquid nicotine formulation has a combined water and water-miscible polyhydric alcohol content of greater than or equal to about 70 percent by weight. More preferably, the carbonated liquid nicotine formulation has a combined water and water-miscible polyhydric alcohol content of greater than or equal to 75 percent by weight. For example, the carbonated liquid nicotine formulation may have a combined water and water-miscible polyhydric alcohol content of greater than or equal to about 80 percent by weight or greater than or equal to about 85 percent by weight.

In embodiments in which the carbonated liquid nicotine formulation comprises water and one or more water-miscible polyhydric alcohols, the ratio of the weight percent water-miscible polyhydric alcohol content to the weight percent water content of the carbonated liquid nicotine formulation may be greater than or equal to about 1 or greater than or equal to about 1.5.

In embodiments in which the carbonated liquid nicotine formulation comprises water and one or more water-miscible polyhydric alcohols, preferably the ratio of the weight percent water-miscible polyhydric alcohol content to the weight percent water content of the carbonated liquid nicotine formulation is greater than or equal to about 2. For example, the ratio of the weight percent water-miscible polyhydric alcohol content to the weight percent water content of the carbonated liquid nicotine formulation may be greater than or equal to about 2.5, greater than or equal to about 3, greater than or equal to about 3 or greater than or equal to about 3.5.

Most preferably, the carbonated liquid nicotine formulation comprises water and one or more water-miscible polyhydric alcohols selected from the group consisting of glycerine and propylene glycol.

According to a more preferred embodiment of the invention there is provided a carbonated liquid nicotine formulation for use in an aerosol-generating system, the carbonated liquid nicotine formulation comprising water and one or more water-miscible polyhydric alcohols selected from the group consisting of glycerine and propylene glycol, wherein the carbonated liquid formulation has an ethanol content of less than 1 percent by weight, a nicotine content of greater than or equal to 0.25 percent by weight and a carbon dioxide content of greater than or equal to 0.5 percent by weight.

According to a more preferred embodiment of the invention there is also provided a cartridge for use in an aerosol-generating system, the cartridge containing a carbonated liquid nicotine formulation comprising water and one or more water-miscible polyhydric alcohols selected from the group consisting of glycerine and propylene glycol, wherein the carbonated liquid formulation has an ethanol content of less than 1 percent by weight, a nicotine content of greater than or equal to 0.25 percent by weight and a carbon dioxide content of greater than or equal to 0.5 percent by weight.

According to a more preferred embodiment of the invention there is further provided an aerosol-generating system comprising: a carbonated liquid nicotine formulation comprising water and one or more water-miscible polyhydric alcohols selected from the group consisting of glycerine and propylene glycol, wherein the carbonated liquid formulation has an ethanol content of less than 1 percent by weight, a nicotine content of greater than or equal to 0.25 percent by weight and a carbon dioxide content of greater than or equal to 0.5 percent by weight; and an atomiser configured to generate an aerosol from the carbonated liquid nicotine formulation.

In embodiments in which the carbonated liquid nicotine formulation comprises water and one or more water-miscible polyhydric alcohols selected from the group consisting of glycerine and propylene glycol, preferably the carbonated liquid nicotine formulation has a combined water, glycerine and propylene glycol content of greater than or equal to about 70 percent by weight. More preferably, the carbonated liquid nicotine formulation has a combined water, glycerine and propylene glycol content of greater than or equal to 75 percent by weight. For example, the carbonated liquid nicotine formulation may have a combined water, glycerine and propylene glycol content of greater than or equal to about 80 percent by weight or greater than or equal to about 85 percent by weight.

In embodiments in which the carbonated liquid nicotine formulation comprises water and one or more water-miscible polyhydric alcohols selected from the group consisting of glycerine and propylene glycol, the ratio of the weight percent combined glycerine and propylene glycol content to the weight percent water content of the carbonated liquid nicotine formulation may be greater than or equal to about 1 or greater than or equal to about 1.5.

In embodiments in which the carbonated liquid nicotine formulation comprises water and one or more water-miscible polyhydric alcohols selected from the group consisting of glycerine and propylene glycol, preferably the ratio of the weight percent combined glycerine and propylene glycol content to the weight percent water content of the carbonated liquid nicotine formulation is greater than or equal to about 2. For example, the ratio of the weight percent combined glycerine and propylene glycol content to the weight percent water content of the carbonated liquid nicotine formulation may be greater than or equal to about 2.5, greater than or equal to about 3, greater than or equal to about 3 or greater than or equal to about 3.5.

The liquid nicotine formulation may comprise one or more flavourants. Suitable flavourants include, but are not limited to, menthol.

Preferably, the liquid nicotine formulation has a flavourant content of less than or equal to about 4 percent by weight. More preferably, the liquid nicotine formulation has a flavourant content of less than or equal to about 3 percent by weight.

The nicotine may be a nicotine salt.

The nicotine salt may comprise a carboxylic acid.

Preferably, the nicotine salt may comprise 2-ethylbutyric acid, fumaric acid, lactic acid, acetic acid, succinic acid, sorbic acid (2E,4E), myristic acid, hexanoic acid, oleic acid (Z-), adipic acid, propanoic acid, cinnamic acid, undecanoic acid, octanoic acid, benzoic acid, hydrochloric acid, malic acid, levulinic acid, formic acid, pyruvic acid, tartaric acid (L-), citric acid, aconitic acid, butyric acid, decanoic acid, isobutyric acid, isovaleric acid, 2-methylbutyric acid, nonanoic acid, phenylacetic acid, salicylic acid, phosphoric acid, cyclohexylacetic acid, lauric acid, palmitic acid, stearic acid, linoleic acid, cyclohexanecarboxylic acid, crotonic acid, vanillic acid, valeric acid, cycloheptanecarboxylic acid, tartronic acid (2-hydroxymalonic acid), glycolic acid, oxalic acid, dibenzoyl-L-tartaric acid, oleic acid (E-), deoxycholic acid, or combinations thereof.

Preferably, the nicotine salt may comprise lactic acid, levulinic acid, fumaric acid, benzoic acid, or combinations thereof.

The ratio of the weight percent carboxylic acid content to the weight percent nicotine content of the nicotine salt may be 1.

The ratio of the weight percent carboxylic acid content to the weight percent nicotine content of the nicotine salt may be greater than or equal to 0.5. The ratio of the weight percent carboxylic acid content to the weight percent nicotine content of the nicotine salt may be greater than or equal to 0.75. The ratio of the weight percent carboxylic acid content to the weight percent nicotine content of the nicotine salt may be greater than or equal to 0.8. The ratio of the weight percent carboxylic acid content to the weight percent nicotine content of the nicotine salt may be greater than or equal to 0.9. The ratio of the weight percent carboxylic acid content to the weight percent nicotine content of the nicotine salt may be greater than or equal to 1. The ratio of the weight percent carboxylic acid content to the weight percent nicotine content of the nicotine salt may be greater than or equal to 1.1. The ratio of the weight percent carboxylic acid content to the weight percent nicotine content of the nicotine salt may be greater than or equal to 1.2. The ratio of the weight percent carboxylic acid content to the weight percent nicotine content of the nicotine salt may be greater than or equal to 1.25. The ratio of the weight percent carboxylic acid content to the weight percent nicotine content of the nicotine salt may be greater than or equal to 1.5.

The ratio of the weight percent carboxylic acid content to the weight percent nicotine content of the nicotine salt may be less than 0.5. The ratio of the weight percent carboxylic acid content to the weight percent nicotine content of the nicotine salt may be less than 0.75. The ratio of the weight percent carboxylic acid content to the weight percent nicotine content of the nicotine salt may be less than 0.8. The ratio of the weight percent carboxylic acid content to the weight percent nicotine content of the nicotine salt may be less than 0.9. The ratio of the weight percent carboxylic acid content to the weight percent nicotine content of the nicotine salt may be less than 1. The ratio of the weight percent carboxylic acid content to the weight percent nicotine content of the nicotine salt may be less than 1.1. The ratio of the weight percent carboxylic acid content to the weight percent nicotine content of the nicotine salt may be less than 1.2. The ratio of the weight percent carboxylic acid content to the weight percent nicotine content of the nicotine salt may be less than 1.25. The ratio of the weight percent carboxylic acid content to the weight percent nicotine content of the nicotine salt may be less than 1.5.

The ratio of the weight percent carboxylic acid content to the weight percent nicotine content of the nicotine salt may be between 0.5 and 1.5. The ratio of the weight percent carboxylic acid content to the weight percent nicotine content of the nicotine salt may be between 0.75 and 1.5. The ratio of the weight percent carboxylic acid content to the weight percent nicotine content of the nicotine salt may be between 0.75 and 1.25. The ratio of the weight percent carboxylic acid content to the weight percent nicotine content of the nicotine salt may be between 0.8 and 1.2. The ratio of the weight percent carboxylic acid content to the weight percent nicotine content of the nicotine salt may be between 0.9 and 1.2. The ratio of the weight percent carboxylic acid content to the weight percent nicotine content of the nicotine salt may be between 0.9 and 1.1. The ratio of the weight percent carboxylic acid content to the weight percent nicotine content of the nicotine salt may be between 0.9 and 1. The ratio of the weight percent carboxylic acid content to the weight percent nicotine content of the nicotine salt may be between 1 and 1.1.

According to the invention there is also provided a cartridge for use in an aerosol-generating system, the cartridge containing a carbonated liquid nicotine formulation according to the invention.

The cartridge may comprise an atomiser configured to generate an aerosol from the carbonated liquid nicotine formulation.

A cartridge containing the liquid nicotine formulation and an atomiser configured to generate an aerosol from the carbonated liquid nicotine formulation may be referred to as a "cartomiser".

The atomiser may be a thermal atomiser.

As used herein with reference to the invention, the term "thermal atomiser" describes an atomiser that is configured to heat the carbonated liquid nicotine formulation to generate an aerosol.

The cartridge may comprise any suitable type of thermal atomiser.

The thermal atomiser may comprise a heater and a liquid transport element configured to transport carbonated liquid nicotine formulation to the heater.

The liquid transport element may comprise a capillary wick. The heater may comprise a heater coil.

The thermal atomiser may comprise an electric heater. For example, the thermal atomiser may comprise an electric heater comprising a resistive heating element or an inductive heating element.

The atomiser may be a non-thermal atomiser.

As used herein with reference to the invention, the term "non-thermal atomiser" describes an atomiser that is configured to generate an aerosol from the carbonated liquid nicotine formulation by means other than heating.

The cartridge may comprise any suitable type of non-thermal atomiser.

For example, the non-thermal atomiser may be an impinging jet atomiser, an ultrasonic atomiser or a vibrating mesh atomiser.

According to the invention there is further provided an aerosol-generating system comprising a carbonated liquid nicotine formulation according to the invention and an atomiser configured to generate an aerosol from the liquid nicotine formulation.

The atomiser may be a thermal atomiser.

The aerosol-generating system may comprise any suitable type of thermal atomiser.

The thermal atomiser may comprise a heater and a liquid transport element configured to transport carbonated liquid nicotine formulation to the heater.

The liquid transport element may comprise a capillary wick. The heater may comprise a heater coil.

The thermal atomiser may comprise an electric heater. For example, the thermal atomiser may comprise an electric heater comprising a resistive heating element or an inductive heating element.

The atomiser may be a non-thermal atomiser.

The aerosol-generating system may comprise any suitable type of non-thermal atomiser.

For example, the non-thermal atomiser may be an impinging jet atomiser, an ultrasonic atomiser or a vibrating mesh atomiser.

The aerosol-generating system may comprise: a cartridge according to the invention containing the carbonated liquid nicotine formulation; and an aerosol-generating device comprising a housing defining a device cavity configured to receive at least a portion of the cartridge.

The aerosol-generating system may comprise: a consumable cartridge according to the invention containing the carbonated liquid nicotine formulation; and a reusable aerosol-generating device comprising a housing defining a device cavity configured to receive at least a portion of the cartridge.

The aerosol-generating device may comprise a battery and control electronics.

The aerosol-generating system may comprise: a cartridge according to the invention containing the carbonated liquid nicotine formulation and the atomiser; and an aerosol-generating device comprising a housing defining a device cavity configured to receive at least a portion of the cartridge.

The aerosol-generating system may comprise: a cartridge according to the invention containing the carbonated liquid nicotine formulation; and an aerosol-generating device comprising a housing defining a device cavity configured to receive at least a portion of the cartridge and the atomiser.

For the avoidance of doubt, features described above in relation to the carbonated liquid nicotine formulation of the invention may also relate, where appropriate, to the cartridge of the invention and the aerosol-generating system of the invention. Similarly, features described above in relation to the cartridge of the invention may also relate, where appropriate, to the aerosol-generating system of the invention, and *vice versa.*

The invention will now be described, by way of example only, with reference to the following example and accompanying drawings, in which:
Figure 1a shows a schematic illustration of an aerosol-generating system comprising an aerosol-generating device and a cartridge being inserted into the aerosol-generating device;
Figure 1b shows a schematic illustration of the aerosol-generating system of Figure 1a, in which the cartridge is received in the aerosol-generating device; and
Figure 2 shows the nicotine yield of aerosols generated from the liquid nicotine compositions shown in Table 1 using an IQOS MESH^{™} device.

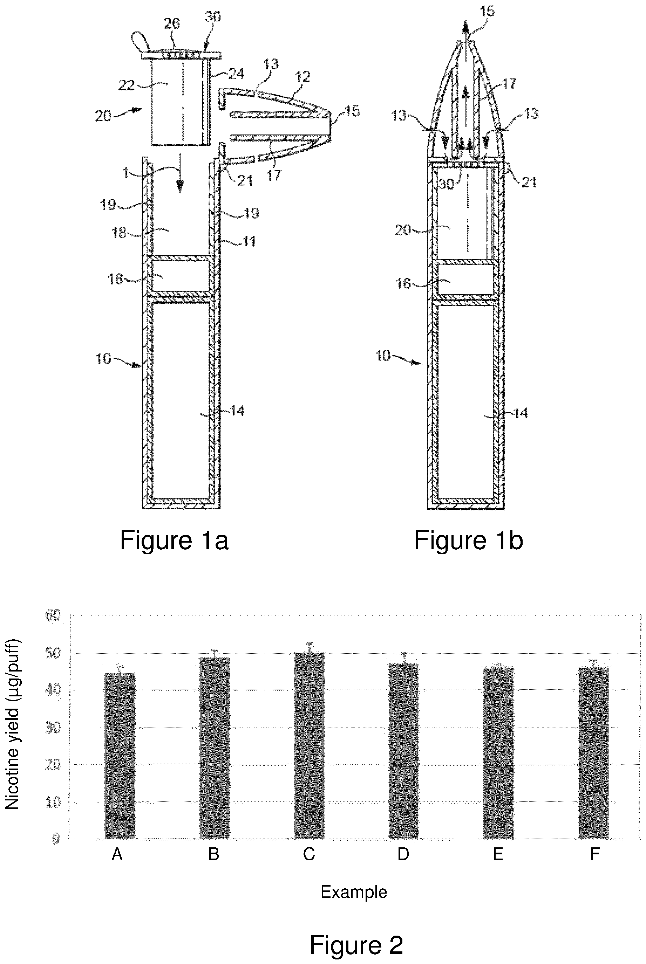

Figures 1a and 1b are schematic illustrations of an exemplary aerosol-generating system according to the invention. The aerosol-generating system shown in Figures 1a and 1b is like that disclosed in WO 2015/117702 A1 and comprises an aerosol-generating device 10 and a cartridge 20.

The aerosol-generating device 10 is portable and has a size comparable to a conventional cigar or cigarette. The aerosol-generating device 10 comprises a main body 11 and a mouthpiece portion 12. The main body 11 contains a battery 14 and control electronics 16. The main body 11 defines a device cavity 18 configured to receive the cartridge 20.

The device cavity 18 is of substantially circular transverse cross-section and is sized to receive the cartridge 20. Electrical connectors 19 are provided at the sides of the device cavity 18 to provide an electrical connection between the control electronics 16 and the battery 14 and corresponding electrical contacts provided on the cartridge 20.

The mouthpiece portion 12 is connected to the main body 11 by a hinged connection 21 and can move between an open position as shown in Figure 1a and a closed position as shown in Figure 1b. The mouthpiece portion 12 is placed in the open position to allow for insertion and removal of the cartridge 20 from the aerosol-generating device 10 and is placed in the closed position when the aerosol-generating system is to be used to generate an aerosol, as described below. The mouthpiece portion 12 comprises a plurality of air inlets 13 and an outlet 15. In use, a user draws on the outlet 15 to draw air into the mouthpiece portion 12 through the air inlets 13, through the mouthpiece portion to the outlet 15, and thereafter into the mouth and lungs of the user. As shown in Figures 1a and 1b, internal baffles 17 are provided to force the air flowing through the mouthpiece portion 12 past the cartridge 20.

Figure 1a shows the cartridge 20 just prior to insertion into the aerosol-generating device 10, with the arrow 1 in Figure 1a indicating the direction of insertion of the cartridge 20.

The cartridge 20 contains a carbonated liquid nicotine formulation according to the invention. The cartridge 20 is replaceable by a user when the carbonated liquid nicotine formulation contained in the cartridge 20 is depleted. The cartridge 20 comprises a generally circular cylindrical housing 24 that has a size and shape selected to be received into the device cavity 18. The housing 24 contains a capillary material (not shown) that is soaked in the carbonated liquid nicotine formulation. In this example the carbonated liquid nicotine formulation comprises 17 percent by weight water, 40 percent by weight glycerine, 40 percent by weight propylene glycol, 2 percent by weight carbon dioxide and 1 percent by weight nicotine. A capillary material is a material that actively conveys liquid from one end to another, and may be formed from any suitable material. In this example the capillary material is formed from polyester.

The housing 24 has an open end to which an atomiser comprising a heater assembly 30 is fixed. The heater assembly 30 comprises a substrate having an aperture formed in it, a pair of electrical contacts fixed to the substrate and separated from each other by a gap, and a plurality of electrically conductive heater filaments spanning the aperture and fixed to the electrical contacts on opposite sides of the aperture.

The heater assembly 30 is covered by a removable cover 26 that is secured to the heater assembly 30. The removable cover 26 is liquid impermeable and is configured to be removed from the heater assembly 30 by a user prior to use of the aerosol-generating system. The removable cover 26 may be secured to the heater assembly 30 by any suitable means. Suitable means include, but are not limited to, adhesive bonding, thermal bonding, such as for example, laser bonding and ultrasonic welding, and combinations thereof. As shown in Figure 1a, the removable cover 26 is provided with a pull tab to facilitate removal of the removable cover 26 by a user prior to use of the aerosol-generating system.

In use, with the mouthpiece portion 12 of the aerosol-generating device 10 in the open position shown in Figure 1a, the cartridge 20 is inserted into the device cavity 18 of the aerosol-generating device 10 and the removable cover 26 is removed from the cartridge 10. Once the cover 26 has been removed, the heater filaments are exposed through the aperture in the substrate so that vaporised carbonated liquid nicotine formulation can escape into the air flow past the heater assembly 30.

In this position, the electrical connectors 19 provided at the sides of the device cavity 18 rest against the electrical contacts provided on the cartridge 20. The mouthpiece portion 12 is then moved to the closed position shown in Figure 1b. The mouthpiece portion 12 is retained in the closed position by a clasp mechanism (not shown).

In the closed position the mouthpiece portion 12 of the aerosol-generating device 10 retains the cartridge 20 in electrical contact with the electrical connectors 19 provided at the sides of the device cavity 18 so that, in use, a good electrical connection is maintained regardless of the orientation of the aerosol-generating system. The mouthpiece portion 12 may include an annular elastomeric element that engages a surface of the cartridge 20 and is compressed between a rigid element of the mouthpiece portion 12 and the cartridge 20 when the mouthpiece portion 12 is in the closed position. This may ensure that a good electrical connection is maintained despite manufacturing tolerances. Other mechanisms for maintaining a good electrical connection between the cartridge and the device may be employed.

In use the heater assembly 30 operates by resistive heating. Current is passed through the heater filaments under the control of control electronics 16, to heat the heater filaments to within a desired temperature range.

The air flow through the mouthpiece portion 12 when the aerosol-generating system is used is illustrated in Figure 1d. The mouthpiece portion 12 includes internal baffles 17, which are integrally moulded with the external walls of the mouthpiece portion and ensure that, as air is drawn from the air inlets 13 to the outlet 15, it flows over the heater assembly 30 on the cartridge 10 where carbonated liquid nicotine formulation is being vaporised. As the air passes the heater assembly 30, vaporised carbonated liquid nicotine formulation is entrained in the airflow and cools to form an aerosol before exiting through the outlet 15. Accordingly, in use, the carbonated liquid nicotine formulation passes through the heater assembly 30 by passing through interstices between the heater filaments as it is vaporised.

### Example

An ethanol-free, non-carbonated liquid nicotine formulation not according to the invention (Example A), an ethanol-free, carbonated liquid nicotine formulation according to the invention (Example B), two ethanol-containing, non-carbonated liquid nicotine formulations not according to the invention (Examples C and E) and two ethanol-containing, carbonated liquid nicotine formulations not according to the invention (Examples D and F) are prepared having the compositions shown in Table 1:

**Table 1**

| **Example** | **A** | **B** | **C** | **D** | **E** | **F** |
|---|---|---|---|---|---|---|
| Nicotine (% by weight) | 1.1 | 1.1 | 1.1 | 1.1 | 1.1 | 1.1 |
| Water (% by weight) | 19.6 | 19.2 | 17.6 | 17.3 | 14.7 | 14.5 |
| Vegetable Glycerine (% by weight) | 39.2 | 38.5 | 39.2 | 38.5 | 39.2 | 38.5 |
| Propylene Glycol (% by weight) | 39.2 | 38.5 | 39.2 | 38.5 | 39.2 | 38.5 |
| Carbon Dioxide (% by weight) | 0 | 1.8 | 0 | 1.8 | 0 | 1.7 |
| Ethanol (% by weight) | 0 | 0 | 2 | 1.9 | 4.9 | 4.8 |
| Flavourant (% by weight) | 0.9 | 0.9 | 0.9 | 0.9 | 0.9 | 0.9 |

The liquid nicotine formulations of Examples B, D and F are carbonated using a sodastream^{®} SPIRIT^{™} Sparling Water Maker by:
(i) placing 20g of the non-carbonated liquid nicotine formulation cooled to a temperature between 0 degrees Celsius and 4 degrees Celsius in a 1 litre sodastream^{®} carbonating bottle;
(ii) immersing a plastic tube connected to the carbonating tube of the sodastream^{®} SPIRIT^{™} Sparling Water Maker in the non-carbonated liquid nicotine formulation in the 1 litre sodastream^{®} carbonating bottle;
(iii) depressing the carbonating button of the sodastream^{®} SPIRIT^{™} Sparling Water Maker for around two seconds, releasing and repeating five times to carbonate the liquid nicotine formulation; and
(iv) releasing the pressure in the bottle and then closing the 1 litre sodastream^{®} carbonating bottle containing the carbonated liquid nicotine formulation with a sodastream^{®} fizz-preserving cap.

The quantity of carbon dioxide in each of the carbonated liquid nicotine formulations is calculated by weighing the 1 litre sodastream^{®} carbonating bottle, non-carbonated liquid nicotine formulation, plastic tube and sodastream^{®} fizz-preserving cap prior to step (iii) and weighing the 1 litre sodastream^{®} carbonating bottle, carbonated liquid nicotine formulation, plastic tube and sodastream^{®} fizz-preserving cap after step (iv).

Aerosols are generated from ethanol-free, non-carbonated liquid nicotine formulation not according to the invention (Example A), an ethanol-free, carbonated liquid nicotine formulation according to the invention (Example B), two ethanol-containing, non-carbonated liquid nicotine formulations not according to the invention (Examples C and E) and two ethanol-containing, carbonated liquid nicotine formulations not according to the invention (Examples D and F) using an IQOS MESH^{™} device. Aerosols generated from three VEEV flavor caps containing each liquid nicotine formation were analysed using FT-IR and the average nicotine yield per puff for 10 puffs calculated. The results are shown in Figure 2.

As shown in Figure 2, the average nicotine yield per puff for the aerosols generated from the ethanol-free, carbonated liquid nicotine formulation according to the invention (Example B) is advantageously about 9% greater than the average nicotine yield per puff for the aerosols generated from the ethanol-free, non-carbonated liquid nicotine formulation not according to the invention (Example A).

As also shown in Figure 2, the average nicotine yield per puff for the aerosols generated from the two ethanol-containing, carbonated liquid nicotine formulations not according to the invention (Examples D and F) is not increased compared to the two ethanol-containing, non-carbonated liquid nicotine formulations not according to the invention (Examples C and E).

The data shown in Figure 2 illustrates that carbonation of substantially ethanol-free, liquid nicotine formulations having an ethanol content of less than 1 percent by weight in accordance with the invention advantageously increases the nicotine delivery to a user of inhalable aerosols generated from the liquid nicotine formulations. The data shown in Figure 2 also illustrates that carbonation of liquid nicotine formulations having an ethanol content of greater than 1 percent by weight does not increase the nicotine delivery to a user of inhalable aerosols generated from the liquid nicotine formulations.

## Claims

1. A carbonated liquid nicotine formulation for use in an aerosol-generating system, the carbonated liquid nicotine formulation comprising at least one of water and one or more water-miscible solvents, wherein the carbonated liquid formulation has an ethanol content of less than 1 percent by weight, a nicotine content of greater than or equal to 0.25 percent by weight and a carbon dioxide content of greater than or equal to 0.5 percent by weight.

2. A carbonated liquid nicotine formulation according to claim 1 having an ethanol content of less than or equal to about 0.5 percent by weight.

3. A carbonated liquid nicotine formulation according to claim 1 or 2 having a carbon dioxide content of less than or equal to about 5 percent by weight.

4. A carbonated liquid nicotine formulation according to any one of claims 1 to 3 having a carbon dioxide content of between 1 percent by weight and 3 percent by weight.

5. A carbonated liquid nicotine formulation according to any of claims 1 to 4 having a nicotine content of between 0.5 percent by weight and 5 percent by weight.

6. A carbonated liquid nicotine formulation according to any one of claims 1 to 5 having a total water and water-miscible solvent content of greater than or equal to about 70 percent by weight.

7. A carbonated liquid nicotine formulation according to any one of claims 1 to 6 comprising water and one or more other water-miscible solvents.

8. A carbonated liquid nicotine formulation according to any one of claims 1 to 7 wherein the one or more water-miscible solvents are one or more water-miscible polyhydric alcohols selected from the group consisting of 1 ,3-butanediol, glycerine, propylene glycol, and triethylene glycol.

9. A carbonated liquid nicotine formulation according to any one of claims 1 to 8 wherein the one or more water-miscible solvents are one or more water-miscible polyhydric alcohols selected from the group consisting of glycerine and propylene glycol.

10. A carbonated liquid nicotine formulation according to any one of claims 1 to 9 having a combined glycerine and propylene glycol content of between 25 percent by weight and 85 percent by weight.

11. A carbonated liquid nicotine formulation according to any one of claims 1 to 10 further comprising one or more flavourants.

12. A carbonated liquid nicotine formulation according to claim 11 having a flavourant content of less than or equal to about 4 percent by weight.

13. A cartridge for use in an aerosol-generating system, the cartridge containing a carbonated liquid nicotine formulation according to any one of claims 1 to 12.

14. A cartridge according to claim 13 comprising an atomiser configured to generate an aerosol from the carbonated liquid nicotine formulation.

15. An aerosol-generating system comprising:
a carbonated liquid nicotine formulation according to any one of claims 1 to 12; and
an atomiser configured to generate an aerosol from the carbonated liquid nicotine formulation.

## Patentansprüche

1. Karbonisierte flüssige Nikotinformulierung zum Gebrauch in einem Aerosolerzeugungssystem, wobei die karbonisierte flüssige Nikotinformulierung wenigstens eines von Wasser und einem oder mehreren mit Wasser mischbaren Lösungsmitteln umfasst, wobei die karbonisierte flüssige Formulierung einen Ethanolgehalt von weniger als 1 Gewichtsprozent, einen Nikotingehalt von mehr als oder gleich 0,25 Gewichtsprozent und einen Kohlendioxidgehalt von mehr als oder gleich 0,5 Gewichtsprozent aufweist.

2. Karbonisierte flüssige Nikotinformulierung nach Anspruch 1, aufweisend einen Ethanolgehalt von weniger als oder gleich etwa 0,5 Gewichtsprozent.

3. Karbonisierte flüssige Nikotinformulierung nach Anspruch 1 oder 2, aufweisend einen Kohlendioxidgehalt von weniger als oder gleich etwa 5 Gewichtsprozent.

4. Karbonisierte flüssige Nikotinformulierung nach einem der Ansprüche 1 bis 3, aufweisend einen Kohlendioxidgehalt zwischen 1 Gewichtsprozent und 3 Gewichtsprozent.

5. Karbonisierte flüssige Nikotinformulierung nach einem der Ansprüche 1 bis 4, aufweisend einen Nikotingehalt zwischen 0,5 Gewichtsprozent und 5 Gewichtsprozent.

6. Karbonisierte flüssige Nikotinformulierung nach einem der Ansprüche 1 bis 5, aufweisend einen Gesamtgehalt an Wasser und mit Wasser mischbaren Lösungsmitteln von mehr als oder gleich etwa 70 Gewichtsprozent.

7. Karbonisierte flüssige Nikotinformulierung nach einem der Ansprüche 1 bis 6, aufweisend Wasser und ein oder mehrere andere mit Wasser mischbare Lösungsmittel.

8. Karbonisierte flüssige Nikotinformulierung nach einem der Ansprüche 1 bis 7, wobei das eine oder die mehreren mit Wasser mischbaren Lösungsmittel ein oder mehrere mit Wasser mischbare mehrwertige Alkohole sind, ausgewählt aus der Gruppe bestehend aus 1,3-Butandiol, Glycerin, Propylenglykol und Triethylenglykol.

9. Karbonisierte flüssige Nikotinformulierung nach einem der Ansprüche 1 bis 8, wobei das eine oder die mehreren mit Wasser mischbaren Lösungsmittel ein oder mehrere mit Wasser mischbare mehrwertige Alkohole sind, ausgewählt aus der Gruppe bestehend aus Glycerin und Propylenglykol.

10. Karbonisierte flüssige Nikotinformulierung nach einem der Ansprüche 1 bis 9, aufweisend einen kombinierten Glycerin- und Propylenglykolgehalt zwischen 25 Gewichtsprozent und 85 Gewichtsprozent.

11. Karbonisierte flüssige Nikotinformulierung nach einem der Ansprüche 1 bis 10, ferner aufweisend einen oder mehrere Geschmacksstoffe.

12. Karbonisierte flüssige Nikotinformulierung nach Anspruch 11, aufweisend einen Geschmacksstoffgehalt von weniger als oder gleich etwa 4 Gewichtsprozent.

13. Patrone zum Gebrauch in einem Aerosolerzeugungssystem, wobei die Patrone eine karbonisierte flüssige Nikotinformulierung nach einem beliebigen der Ansprüche 1 bis 12 enthält.

14. Patrone nach Anspruch 13, umfassend einen Zerstäuber, ausgelegt zum Erzeugen eines Aerosols aus der karbonisierten flüssigen Nikotinformulierung.

15. Aerosolerzeugungssystem, aufweisend:
eine karbonisierte flüssige Nikotinformulierung nach einem der Ansprüche 1 bis 12; und
einen Zerstäuber, ausgelegt zum Erzeugen eines Aerosols aus der karbonisierten flüssigen Nikotinformulierung.

## Revendications

1. Formulation de nicotine liquide gazéifiée destinée à être utilisée dans un système de génération d'aérosol, la formulation de nicotine liquide gazéifiée comprenant au moins l'un parmi l'eau et un ou plusieurs solvants miscibles dans l'eau, la formulation liquide gazéifiée ayant une teneur en éthanol inférieure à 1 pour cent en poids, une teneur en nicotine supérieure ou égale à 0,25 pour cent en poids et une teneur en dioxyde de carbone supérieure ou égale à 0,5 pour cent en poids.

2. Formulation de nicotine liquide gazéifiée selon la revendication 1, ayant une teneur en éthanol inférieure ou égale à environ 0,5 pour cent en poids.

3. Formulation de nicotine liquide gazéifiée selon la revendication 1 ou 2, ayant une teneur en dioxyde de carbone inférieure ou égale à environ 5 pour cent en poids.

4. Formulation de nicotine liquide gazéifiée selon l'une quelconque des revendications 1 à 3, ayant une teneur en dioxyde de carbone d'entre 1 pour cent en poids et 3 pour cent en poids.

5. Formulation de nicotine liquide gazéifiée selon l'une quelconque des revendications 1 à 4, ayant une teneur en nicotine d'entre 0,5 pour cent en poids et 5 pour cent en poids.

6. Formulation de nicotine liquide gazéifiée selon l'une quelconque des revendications 1 à 5, ayant une teneur totale en eau et en solvant miscible dans l'eau supérieure ou égale à environ 70 pour cent en poids.

7. Formulation de nicotine liquide gazéifiée selon l'une quelconque des revendications 1 à 6, comprenant de l'eau et un ou plusieurs solvants miscibles dans l'eau.

8. Formulation de nicotine liquide gazéifiée selon l'une quelconque des revendications 1 à 7, dans laquelle les un ou plusieurs solvants miscibles dans l'eau sont un ou plusieurs polyalcools miscibles dans l'eau choisis dans le groupe constitué par 1,3-butanediol, glycérine, propylène glycol et triéthylène glycol.

9. Formulation de nicotine liquide gazéifiée selon l'une quelconque des revendications 1 à 8, dans laquelle les un ou plusieurs solvants miscibles dans l'eau sont un ou plusieurs polyalcools miscibles dans l'eau choisis dans le groupe constitué par glycérine et propylène glycol.

10. Formulation de nicotine liquide gazéifiée selon l'une quelconque des revendications 1 à 9, ayant une teneur combinée en glycérine et propylène glycol d'entre 25 pour cent en poids et 85 pour cent en poids.

11. Formulation de nicotine liquide gazéifiée selon l'une quelconque des revendications 1 à 10, comprenant en outre un ou plusieurs agents aromatisants.

12. Formulation de nicotine liquide gazéifiée selon la revendication 11, ayant une teneur en agent aromatisant inférieure ou égale à environ 4 pour cent en poids.

13. Cartouche destinée à être utilisée dans un système de génération d'aérosol, la cartouche contenant une formulation de nicotine liquide gazéifiée selon l'une quelconque des revendications 1 à 12.

14. Cartouche selon la revendication 13, comprenant un atomiseur configuré pour générer un aérosol à partir de la formulation de nicotine liquide gazéifiée.

15. Système de génération d'aérosol, comprenant :
une formulation de nicotine liquide gazéifiée selon l'une quelconque des revendications 1 à 12 ; et
un atomiseur configuré pour générer un aérosol à partir de la formulation de nicotine liquide gazéifiée.
